# EUROPEAN PATENT APPLICATION

(11) **EP 4 751 730 A1**
(43) Date of publication of application: **03.06.2026**
(21) Application number: 23950452.5
(22) Date of filing: 01.11.2023
(51) Int. Cl.: A61K 38/26, A61K 9/20, A61K 47/10, A61K 47/26, A61K 47/36, A61P 3/10

(54) **FREEZE-DRIED FLASH RELEASE TABLET OF GLP1 POLYPEPTIDE DRUG AND PREPARATION METHOD THEREFOR**

(30) Priority: 28.08.2023 CN 202311090980
(71) Applicant: Panexo Biotech Sg Pte.Ltd, Singapore 409051 (SG)
(72) Inventor: WANG, Yi, Beijing 100141 (CN); DONG, Yanan, Beijing 100141 (CN); LI, Yanfang, Beijing 100141 (CN); WANG, Hongfei, Beijing 100141 (CN); LI, Pengfei, Beijing 100141 (CN)
(74) Representative: Sonnenberg Harrison Partnerschaft mbB
(86) International application number: PCT/CN2023/129027
(87) International publication number: WO 2025/043879

(57) **Abstract**

The present invention provides a freeze-dried orally disintegrating tablet of GLP-1 polypeptide drugs and a preparation method thereof. The freeze-dried orally disintegrating tablet of GLP-1 polypeptide drug comprises, in percentage by mass, 30% to 50% GLP-1 polypeptide-mEV raw solution, 0.1% to 1.0% surfactant, 3% to 5% matrix agent, 1% to 3% binder, 0.01% to 1.0% suspending agent, and water to balance. The GLP-1 polypeptide is selected from the group consisting of liraglutide, semaglutide, and a combination thereof. The present invention further provides a method for preparing a freeze-dried orally disintegrating tablet of GLP-1 polypeptide drug. The orally disintegrating tablet is prepared by a freeze-drying technology, and the resulting product features high drug loading capacity and excellent dissolution effect, which facilitates the oral absorption of the GLP-1 polypeptide. Additionally, the method for preparing the orally disintegrating tablet is straightforward and easy to implement.

## Description

This application claims the priority to Chinese Patent Application No. 202311090980.4, filed with the China National Intellectual Property Administration on August 28, 2023, titled "freeze-dried orally disintegrating tablet of GLP-1 polypeptide drug and preparation method theereof", the entire content of which is incorporated herein by reference.

### FIELD

The present disclosure relates to the field of pharmaceutical preparations, and in particular relates to a freeze-dried orally disintegrating tablet of GLP-1 polypeptide drug and a preparation method thereof.

### BACKGROUND

Glucagon-like peptide-1 (GLP-1) is widely used in the treatment of type 2 diabetes mellitus and severe obesity, with patients administered by injection for a long time. Despite continuous innovations in novel GLP-1 drugs, one enduring challenge remains: patients must rely on lifelong injection therapy. Novo Nordisk's oral semaglutide product was a groundbreaking development in offering an oral GLP-1 therapy. However, the oral bioavailability remains low (only 1%), and the high cost from daily administration places a substantial economic burden on patients. Therefore, in the treatment of type 2 diabetes and severe obesity, there is a significant need for a low-cost, high-efficiency oral delivery technology for GLP-1 drugs in the treatment of type 2 diabetes and severe obesity.

Milk extracellular vesicles (mEVs) are biological nanoparticles formed from phospholipid bilayers that can be extracted in large quantities from milk. Their natural functions include the delivery of bioactive molecules (such as proteins, and small nucleic acids) from the parent (female cow) to the offspring (calf) through the gastrointestinal tract. It has been demonstrated that mEVs are resistant to damage by digestive enzymes in the alimentary canal, low pH and other environmental factors, and are absorbed through the alimentary canal into the bloodstream. Given the widespread consumption of milk, the long-term exposure of most people to mEV (through drinking milk) indicates that mEVs are safe and well-tolerated for oral use in humans. As a result, mEVs have received much attention in recent years as a potential delivery vehicle for an oral drug.

Previous technologies have demonstrated the feasibility of loading small molecule drugs into mEVs. Earlier studies showed that mEVs loaded with GLP-1 (liraglutide) could be absorbed into the bloodstream via the sublingual venous plexus after oral administration. However, to date, there is still no mature solid oral dosage form for mEVs loaded with GLP-1 or for utilizing lipid nanoparticles (LNPs) to load GLP-1 for oral administration and sublingual absorption. Some existing technologies have produced liquid formulations of mEV loaded with liraglutide. Despite improved stability of mEV solutions compared to other types of extracellular vesicles (EVs), maintaining stability of high-concentration mEV solutions remains a technical challenge. Issues such as aggregation, fusion, and adherence to container walls become prominent when the mEV nanoparticle concentration exceeds 1 × 10¹² p/mL. Additionally, some technologies have produced freeze-dried sublingual tablets. However, to maintain mEV activity during the freeze-drying process, large amounts of mannitol or microcrystalline cellulose are required, which can lead to excessively compact tablets that are difficult to disintegrate in the mouth. Moreover, excessive volume increase in the freeze-dried powder makes it challenging to take by mouth at the intended clinical dose.

Typically, the volume of the solution for a single tablet of an appropriate freeze-dried solid dosage form is relatively small, often less than 1 milliliter, whereas the intended clinical dose for mEV oral absorption is 10¹³ particles or even higher. Therefore, there is a need for a concentration process for mEV raw solutions that can achieve concentrations of 1×10¹³ p/mL or even higher, along with a compatible stabilizer formulation.

### SUMMARY

In view of this, a technical problem to be solved by the present disclosure is to provide a orally disintegrating tablet of GLP-1 polypeptide drug that has a high drug concentration, high dissolution efficiency, and facilitates oral sublingual absorption, and a preparation method thereof.

The present disclosure provides a freeze-dried orally disintegrating tablet of GLP-1 polypeptide drug, which comprises, in percentage by mass: 30% to 50% GLP-1 polypeptide-mEV raw solution or GLP-1 polypeptide-lipid nanoparticle (LNP) raw solution, 0.1% to 1.0% surfactant, 3% to 5% matrix agent, 1% to 3% binder, 0.01% to 1% suspending agent, and water to balance.

The surfactant is poloxamer 188.

The present disclosure uses GLP-1 polypeptide-mEV raw solution to prepare the freeze-dried orally disintegrating tablet of GLP-1 polypeptide drug. In addition, it is also possible to prepare the freeze-dried orally disintegrating tablet of GLP-1 polypeptide drug using LNP formed by SM-102, DLin-MC3-DMA, and phospholipids as a carrier.

Further, the matrix agent is selected from mannitol or trehalose; preferably, the matrix agent is trehalose.

Further, the binder is selected from pullulan or gelatin; preferably, the binder is pullulan.

Further, the suspending agent is selected from pullulan or xanthan gum; preferably, the suspending agent is xanthan gum.

Further, the GLP-1 polypeptide-mEV raw solution is prepared from 0 to 1.2 mg of an excipient and 1×10¹⁰ to 1×10¹³ mEV particles per milligram of GLP-1 polypeptide.

The raw materials for preparing the GLP-1 polypeptide-LNP raw solution comprise: per milligram of GLP-1 polypeptide, 0 to 3 mg of an excipient and phospholipid, SM-102 and DLin-MC3-DMA lipid molecules are added.

The excipient is selected from the group consisting of poly-lysine, protamine, poly-arginine, fucoidan and mannitol. Preferably, the excipient is poly-arginine.

In some embodiments, the freeze-dried orally disintegrating tablet of GLP-1 polypeptide drug provided by the present disclosure comprises, in percentage by mass, 50% GLP-1 polypeptide-mEV raw solution or polypeptide-LNP raw solution, 0.2% surfactant, 3% matrix agent, 2% binder, 0.1% suspending agent, and water to balance. In an embodiment of the present disclosure, the mass percentages are calculated based on the mass before freeze-drying.

In the sublingual tablet provided by the present disclosure, the choice of excipients including surfactant, matrix agent, binder and suspending agent is reasonable, so that the prepared sublingual tablet has good disintegration performance and sublingual absorption efficiency. Preliminary experiments have shown that the combination of poloxamer 188, trehalose, pullulan and xanthan gum is more advantageous for maintaining the stability of GLP-1-mEV, enhancing release performance in the mouth, and improving sublingual absorption efficiency compared to other excipient choices. The GLP-1 peptide may be one or more of liraglutide or semaglutide. The GLP-1 polypeptide is selected from the group consisting of liraglutide, semaglutide, and a combination thereof.

In some embodiments, the GLP-1 polypeptide is liraglutide. The freeze-dried orally disintegrating tablet of liraglutide comprises the following raw materials by mass percentage: a 30% to 50% raw solution of liraglutide-mEV or liraglutide-LNP raw solution, 0.1 to 1.0% poloxamer 188, 3% to 5% trehalose, 1% to 3% pullulan, 0.01% to 1% xanthan gum, and water to balance.

In an embodiment, the freeze-dried orally disintegrating tablet of liraglutide comprises, in percentage by mass, 50% liraglutide-mEV raw solution or liraglutide-LNP raw solution, 0.2% poloxamer 188, 3% trehalose, 2% pullulan, 0.1% xanthan gum, and water to balance.

In some embodiments, the GLP-1 polypeptide is semaglutide. The freeze-dried orally disintegrating tablet of semaglutide comprises, in percentage by mass, 30% to 50% semaglutide-mEV raw solution or semaglutide-LNP raw solution, 0.1 to 1.0% poloxamer 188, 3% to 5% trehalose, 1% to 3% pullulan, 0.01% to 1% xanthan gum, and water to balance.

In an embodiment, the freeze-dried orally disintegrating tablet of semaglutide comprises, in percentage by mass, 50% semaglutide-mEV raw solution or semaglutide-LNP raw solution, 0.2% poloxamer 188, 3% trehalose, 2% pullulan, 0.1% xanthan gum, and water to balance.

The present disclosure further provides a method for preparing the freeze-dried orally disintegrating tablet of GLP-1 polypeptide, comprising:
(1) loading GLP-1 polypeptide into mEV to obtain a GLP-1 polypeptide-mEV raw solution, or loading GLP polypeptide into LNP to obtain a GLP-1 polypeptide-LNP raw solution,
(2) concentrating the GLP-1 polypeptide-mEV raw solution or GLP-1 polypeptide-LNP raw solution by ultrafiltration to obtain a concentrated solution,
(3) mixing the surfactant, matrix agent, binder, suspending agent, sweetener and flavoring agent and adding the concentrated solution to obtain a drug solution,
(4) shearing the drug solution in step (3),
(5) degassing the sheared drug solution in step (4),
(6) filling the degassed drug solution in step (5) into a tray hole,
(7) pre-freezing the filled drug solution to obtain a medicinal product,
(8) freeze-drying the pre-frozen medicinal product with an initial temperature set at -30°C; wherein in the freeze-drying process,
   a cold trap temperature is equal to or less than -40°C, a vacuum degree is equal to or less than 200 ubar,
   a shelf temperature rises from -30°C to -25°C for 5 minutes, maintains at - 25°C for 60 minutes, rises from -25°C to -15°C for 10 minutes, maintains at 15°C for 60 minutes, rises from -15°C to -5°C for 10 minutes, maintains at -5°C for 60 minutes, rises from -5°C to 10°C for 25 minutes, maintains at 10°C for 100 minutes, rises from 10°C to 25°C for 15 minutes, and maintains at 25°C for 60 minutes.

Further, the loading of GLP-1 polypeptide into mEV is carried out by:
mixing a GLP-1 polypeptide solution with an mEV solution, and then performing ultrasonic treatment;
wherein in the GLP-1 polypeptide solution, the solvent is a 20 mM Tris buffer containing 100 mM NaCl, with a pH of 8.0,
in the mEV solution, the solvent is a 20 mM Tris buffer containing 100 mM NaCl, with a pH adjusted to 7.0 to 10.0 using a Na₂CO₃/NaHCO₃ buffer containing glycine, and
the ultrasonic treatment is carried out at 99% power and 5°C for 12 h.

Further, the loading of GLP-1 polypeptide into LNP comprises:
mixing an aqueous solution of GLP-1 with an organic solution of lipid molecules, which self-assemble to form an aqueous dispersion of LNP encapsulating GLP-1.

Further, the concentration of the GLP-1 polypeptide-mEV raw solution or GLP-1 polypeptide-LNP raw solution by ultrafiltration comprises:
dissolving the GLP-1 polypeptide-mEV raw solution or GLP-1 polypeptide-LNP raw solution in PBS buffer containing PEG3350, filtering through a hollow fiber column with a molecular weight cut-off of 750 kDa, and concentrating to 1/5th of a volume of the raw solution (at a flow rate of 405 mL/min). Experiments have shown that the ultrafiltration concentration has an important positive significance in improving the loading capacity of the drug. The present disclosure optimizes the parameters of ultrafiltration concentration to achieve optimal results, thus improving the loading effect even further.

Further, the shearing in step (2) is carried out in an emulsifier;
Further, the degasification in step (3) is carried out in an emulsifier or in a degassing bottle.

Further, the shearing is carried out at a rotational speed of 100 to 3000 rpm for 3 to 20 minutes. Preferably, the emulsifier operates at a rotational speed of 300 rpm for 10 minutes.

Further, the pre-freezing is carried out at -60 to -80°C. Preferably, the pre-freezing is carried out at -60°C.

Further, the pre-freezing is carried out for 10 to 100 minutes. Preferably, the pre-freezing is carried out for 30 minutes.

The present disclosure provides a freeze-dried orally disintegrating tablet of GLP-1 polypeptide drug, which is prepared by a freeze-drying technology, and the resulting product features high drug loading capacity, excellent dissolution effect, and high sublingual absorption efficiency. Additionally, the method for preparing the orally disintegrating tablet is straightforward and easy to implement.

### BRIEF DESCRIPTION OF DRAWINGS

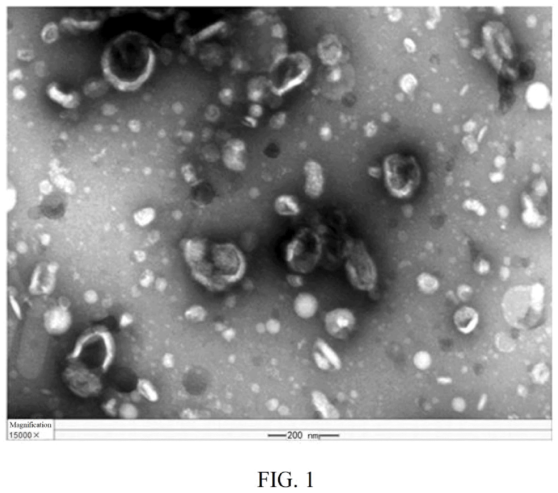

FIG. 1 shows the electron microscopy image of mEV in the absence of any additives.

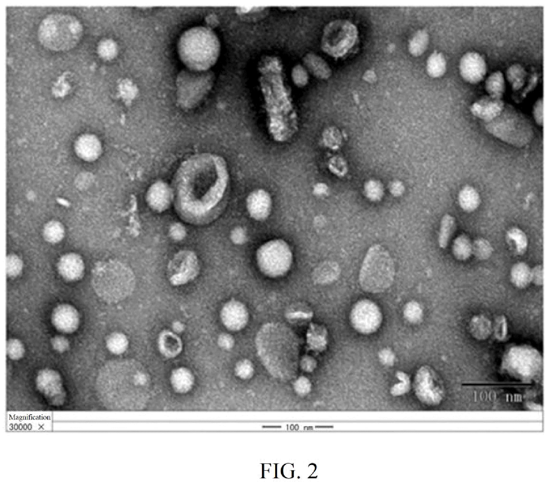

FIG. 2 shows the electron microscopy image of mEV in the presence of 0.5% PEG3350 additive.

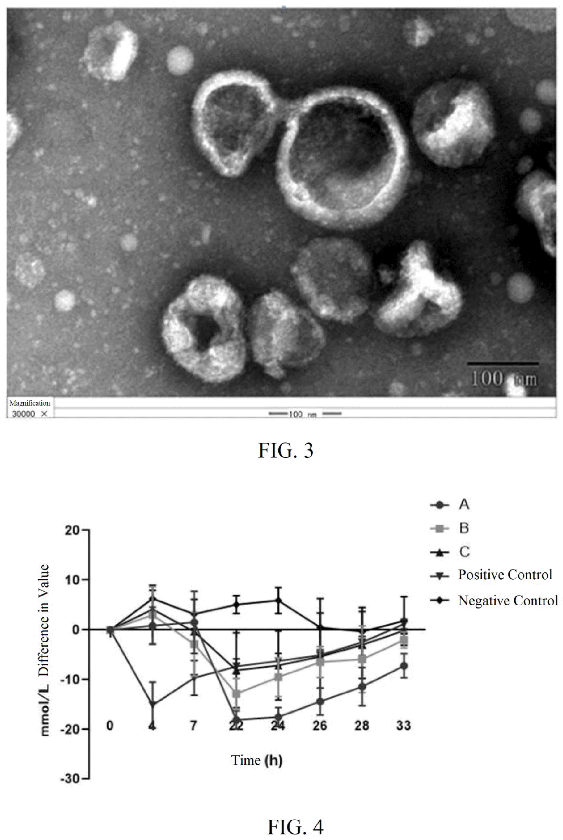

FIG. 3 shows the electron microscopy image of mEV in the presence of 5% PEG3350 additive.
FIG. 4 shows the measurement results of blood glucose concentration in animal pharmacodynamics tests.

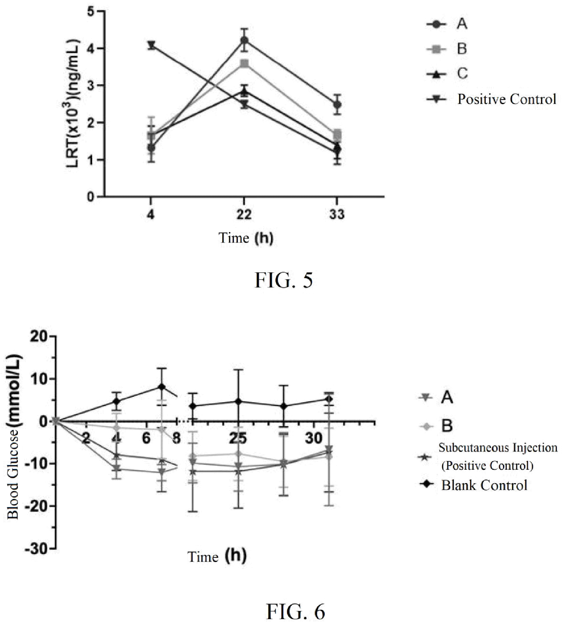

FIG. 5 shows the blood drug concentrations of animals.
FIG. 6 shows the measurement results of blood glucose concentration of mice in each group after administration.

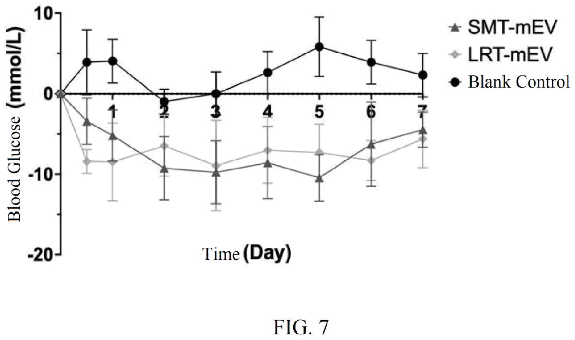

FIG. 7 shows the measurement results of blood glucose concentration after administration of LRT-mEV and SMT-mEV.

### DETAILED DESCRIPTION

In order to better understand the technical content of the present disclosure, the following examples are described in detail for the purpose of better understanding the content of the present disclosure and not to limit the protection scope of the disclosure. The reagents used in the examples of the present disclosure are all commercially available.

### Example 1: Production of High-Concentration mEV Raw Solution Loaded with Liraglutide

Purpose: To load liraglutide (LRT) into mEVs and concentrate the mEV solution using tangential flow ultrafiltration technology while maintaining the stability of particle count and structure of mEVs.

Materials: Milk exosomes (mEVs) prepared with reference to patent 202110097550.X.

### Methods:

### 1. Loading:

### (1) Chemical Loading Method

a. Four 15 mL tubes were used, each containing a total of 7.5E+10 mEVs, which were diluted to a final volume of 2 mL with 20 mM Tris 8.0 + 100 mM NaCl. The pH of the diluted mEV solution was adjusted to 7.0, 8.0, 9.0, and 10.0 using the following buffers: 0.1 M Glycine (pH 7.0), 0.1 M Na₂CO₃/NaHCO₃ (pH 9.04), and 0.1 M Na₂CO₃/NaHCO₃ (pH 10.08). All mEV samples were adjusted to a final volume of 6 mL with the corresponding pH buffer. Each pH-adjusted mEV solution was divided into 15 equal portions, each containing 5.0E+09 mEVs, and designated as mEV solutions.
b. 100 µL of a prepared 0.5-2 mg/mL liraglutide (LRT) solution (dissolved in 20 mM Tris 8.0+100 mM NaCl buffer) was added to each mEV solution containing 5.0E+09 particles with various pH, and mixed well by inverting to obtain a set of LRT-mEV solutions.
c. To each LRT-mEV solution, one of the following additives were added: poly-lysine, protamin, poly-arginine, fucoidan, or mannitol (final concentration ensuring LRT: additive = 1:1).
d. Each sample was placed in a large ultrasonic cleaner, and subjected to ultrasonic treatment at 99% power and 5°C for 12 h.
e. These samples were passed through a Capto Core 700 chromatography column to remove free LRT to obtain a purified LRT-mEV raw solution.

### (2) Osmotic Pressure-Stimulated Loading Method

a. A 50% (m/v) aqueous solution of sucrose was prepared by dissolving 25 g of sucrose granules in 50 mL of ultrapure water. A 0.1×PBS solution of LRT was prepared by dissolving 14.10 mg of powder in 1.41 mL of 0.1 ×PBS, achieving a final concentration of 10 mg/mL.
b. mEV solutions (4E+10 p/mL) with varying sucrose concentrations (0%, 1%, 3%, 5%, 10%, 15%, 20%, and 30% (m/v)) were prepared.
c. The 0.1×PBS solution of LRT was added to the sucrose solution of mEVs until the sucrose concentration in this solution was reduced to 0.2%.
d. Each sample was placed in a large ultrasonic cleaner and subjected to ultrasonic treatment at 99% power and 5°C for 12 h.
e. The samples were passed through a Capto Core 700 chromatography column to remove free LRT to obtain a purified LRT-mEV raw solution.

### 2. Tangential Flow Ultrafiltration Concentration Through Hollow Fiber Columns

### (3) Pre-Treatment:

Preparation of 10% (m/v) PEG3350: 60 mL of 50% PEG3350 stock solution was adjusted to a final volume of 300 mL with water.

The purified LRT-mEV raw solution (450 mL, with a particle concentration of 1.6E+13 p/mL) by the chemical loading method above was divided into three equal portions.

a. One portion, designated as 0% PEG3350-EV, involved mixing 150 mL of LRT-mEV raw solution with 1× PBS to a final volume of 400 mL;
b. Another portion, designated as 0.5% PEG3350-EV, involved mixing 150 mL of LRT-mEV raw solution with 20 mL of 10% (m/v) PEG3350 and adjusting to a final volume of 400 mL with PBS, resulting in a final PEG3350 concentration of 0.5%;
c. The third portion, designated as 5% PEG3350-EV, involved mixing 150 mL of LRT-mEV raw solution with 200 mL of 10% (m/v) PEG3350 and adjusting to a final volume of 400 mL with PBS, resulting in a final PEG3350 concentration of 5%.

### (4) Tangential Flow Ultrafiltration Concentration Through Hollow Fiber Columns

a. Cleaning the Cytiva AKTA FLUX: The hollow fiber column (MWCO = 750 kDa) was cleaned using ultrapure water at a flow rate of 405 mL/min until the filtrate pH was around 7.4.
b. Concentration was initiated at a flow rate of 405 mL/min.
c. The solution was concentrated to 20 mL.
d. 100 mL of PBS buffer was added to wash the filter.
e. The solution was concentrated to 20 mL, and the concentrated liquid was collected.

### 3. Measurement of Particle Count

The particle count of the prepared mEV-LRT samples was measured using Flow NanoAnalyzer from NanoFCM (Xiamen).

### 4. LRT Detection:

The LRT polypeptide content in the prepared mEV-LRT samples was detected with GLP-1 polypeptide monoclonal antibody (ANTIBODYSHOP ABS 046-03/ABS 033-108) by enzyme-linked immunosorbnent assay (ELISA) according to the instructions provided by the antibody manufacturer.

### Results

### 1, Chemical Loading Method:

The loading capacity of LRT per mEV particle was compared under different pH, additives, and LRT stock solution concentrations following low-power ultrasonic treatment. It was observed that at pH 10, mEV exhibited the highest loading capacity for LRT, with approximately 3000 to 20,000 LRT peptide molecules per mEV particle. Overall, mEV demonstrated the best loading efficiency for LRT either with the addition of poly-arginine or without adding additives. Additionally, when different concentrations of LRT stock solutions with equal volume were used for loading, the loading capacity of LRT into mEV increased with higher concentrations of LRT.

**Table 1: Comparison of mEV Loading Capacity for LRT under Different LRT Stock Solution Concentrations, Additive Conditions, and pH Levels**

| Additives | LRT with an initial concentration of 0.5 mg/mL | | | | | | | | | | | |
|---|---|---|---|---|---|---|---|---|---|---|---|---|
| | pH 7.0 | | | pH 8.0 | | | pH 9.0 | | | pH 10.0 | | |
| | LRT content (µg) | Total particle count of mEV (particles) | Loading capacity per particle (particles) | LRT content (µg) | Total particle count of mEV (particles) | Loading capacity per particle (particles) | LRT content (µg) | Total particle count of mEV (particles) | Loading capacity per particle (particles) | LRT content (µg) | Total particle count of mEV (particles) | Loading capacity per particle (particles) |
| Poly-lysine | 3.66E-03 | 6.04E+08 | 9.12E+02 | 2.86E-03 | 2.38E+08 | 1.81E+03 | 3.01E-03 | 2.19E+08 | 2.07E+03 | 3.57E-03 | 1.43E+08 | 3.76E+03 |
| Protamin | 1.51E-03 | 2.73E+08 | 8.32E+02 | 4.71E-03 | 6.13E+08 | 1.16E+03 | 1.03E-02 | 7.35E+08 | 2.11E+03 | 8.69E-03 | 4.86E+08 | 2.69E+03 |
| Poly-arginine | 4.42E-03 | 5.43E+08 | 1.23E+03 | 7.77E-03 | 5.73E+08 | 2.04E+03 | 7.79E-03 | 3.03E+08 | 3.87E+03 | 2.94E-02 | 7.21E+08 | 6.14E+03 |
| Fucoidan | 1.10E-03 | 2.45E+08 | 6.76E+02 | 1.46E-03 | 1.49E+08 | 1.47E+03 | 3.01E-03 | 2.08E+08 | 2.18E+03 | 2.20E-02 | 7.68E+08 | 4.31E+03 |
| Mannitol | 4.00E-03 | 6.08E+08 | 9.91E+02 | 1.76E-03 | 1.57E+08 | 1.69E+03 | 1.14E-02 | 6.90E+08 | 2.49E+03 | 3.82E-03 | 1.61E+08 | 3.57E+03 |
| Additive-free | 2.21E-02 | 1.65E+09 | 2.02E+03 | 1.12E-02 | 7.23E+08 | 2.33E+03 | 2.21E-02 | 7.17E+08 | 4.64E+03 | 6.05E-03 | 1.00E+08 | 9.11E+03 |

| Additives | LRT with an initial concentration of 1.0 mg/mL | | | | | | | | | | | |
|---|---|---|---|---|---|---|---|---|---|---|---|---|
| | pH 7.0 | | | pH 8.0 | | | pH 9.0 | | | pH 10.0 | | |
| | LRT content (µg) | Total particle count of mEV (particles) | Loading capacity per particle (particles) | LRT content (µg) | Total particle count of mEV (particles) | Loading capacity per particle (particles) | LRT content (µg) | Total particle count of mEV (particles) | Loading capacity per particle (particles) | LRT content (µg) | Total particle count of mEV (particles) | Loading capacity per particle (particles) |
| Poly-lysine | 6.92E-03 | 2.91E+08 | 3.58E+03 | 2.26E-02 | 9.03E+08 | 3.77E+03 | 2.00E-02 | 6.80E+08 | 4.42E+03 | 1.79E-02 | 4.24E+08 | 6.36E+03 |
| Protamin | 1.02E-02 | 7.60E+08 | 2.01E+03 | 3.43E-02 | 2.13E+09 | 2.42E+03 | 2.58E-02 | 1.01E+09 | 3.83E+03 | 8.79E-03 | 2.25E+08 | 5.87E+03 |
| Poly-arginine | 2.62E-02 | 9.66E+08 | 4.09E+03 | 3.93E-03 | 1.40E+08 | 4.23E+03 | 8.21E-03 | 2.00E+08 | 6.17E+03 | 5.08E-02 | 1.00E+09 | 7.62E+03 |
| Fucoidan | 1.09E-02 | 8.32E+08 | 1.97E+03 | 4.05E-03 | 2.20E+08 | 2.77E+03 | 5.08E-02 | 2.31E+09 | 3.31E+03 | 4.78E-02 | 1.21E+09 | 5.93E+03 |
| Mannitol | 1.08E-02 | 8.58E+08 | 1.90E+03 | 3.36E-03 | 2.09E+08 | 2.42E+03 | 6.92E-03 | 3.07E+08 | 3.40E+03 | 5.34E-02 | 1.71E+09 | 4.70E+03 |
| Additive-free | 4.26E-02 | 1.51E+09 | 4.24E+03 | 4.16E-02 | 9.41E+08 | 6.66E+03 | 5.06E-02 | 1.08E+09 | 7.08E+03 | 5.47E-02 | 7.21E+08 | 1.14E+04 |

| Additives | LRT with an initial concentration of 2.0 mg/mL | | | | | | | | | | | |
|---|---|---|---|---|---|---|---|---|---|---|---|---|
| | pH 7.0 | | | pH 8.0 | | | pH 9.0 | | | pH 10.0 | | |
| | LRT content (µg) | Total particle count of mEV (particles) | Loading capacity per particle (particles) | LRT content (µg) | Total particle count of mEV (particles) | Loading capacity per particle (particles) | LRT content (µg) | Total particle count of mEV (particles) | Loading capacity per particle (particles) | LRT content (µg) | Total particle count of mEV (particles) | Loading capacity per particle (particles) |
| Poly-lysine | 9.72E-03 | 3.81E+08 | 3.84E+03 | 3.81E-02 | 1.16E+09 | 4.95E+03 | 5.81E-03 | 1.60E+08 | 5.47E+03 | 2.65E-02 | 4.85E+08 | 8.22E+03 |
| Protamin | 1.50E-02 | 8.69E+08 | 2.60E+03 | 3.74E-02 | 1.25E+09 | 4.51E+03 | 3.88E-02 | 1.10E+0% | 5.28E+03 | 1.21E-02 | 2.29E+08 | 7.98E+03 |
| Poly-arginine | 3.50E-02 | 1.18E+09 | 4.48E+03 | 7.07E-02 | 1.39E+09 | 7.67E+03 | 6.15E-02 | 1.08E+09 | 8.61E+03 | 7.51E-02 | 1.15E+09 | 9.86E+03 |
| Fucoidan | 1.60E-02 | 9.81E+08 | 2.46E+03 | 1.02E-02 | 3.50E+08 | 4.40E+03 | 6.29E-02 | 1.73E+09 | 5.48E+03 | 1.30E-02 | 2.58E+08 | 7.59E+03 |
| Mannitol | 1.61E-02 | 9.50E+08 | 2.55E+03 | 3.34E-02 | 1.03E+09 | 4.87E+03 | 2.95E-02 | 7.78E+08 | 5.72E+03 | 7.89E-02 | 1.95E+09 | 6.08E+03 |
| Additive-free | 1.02E-02 | 3.33E+08 | 4.63E+03 | 7.48E-02 | 1.23E+09 | 9.16E+03 | 1.00E-01 | 1.15E+09 | 1.30E+04 | 2.06E-03 | 1.60E+07 | 1.93E+04 |

### 2, Loading results of osmotic pressure stimulation

The loading capacity of LRT per mEV particle was compared under different osmotic pressure stimuli. At a sucrose concentration of 20% w/v, the loading capacity of LRT per mEV particle was highest, with approximately 2600 LRT peptide molecules. However, the loading efficiency of mEV for LRT using this osmotic pressure stimulation method was less effective compared to the chemical loading method.

**Table 2: Comparison of LRT Loading Capacity per mEV Particle under**

| Different Osmotic Pressure Stimuli | | | |
|---|---|---|---|
| Sucrose Concentration (w/v) | LRT content (µg) | Total particle count of mEV (particles) | Loading capacity per particle (particles) |
| 0% | 0.00569 | 1.24E+09 | 6.91E+02 |
| 1% | 0.00473 | 5.94E+08 | 1.20E+03 |
| 3% | 0.00538 | 1.96E+09 | 4.13E+02 |
| 5% | 0.00139 | 1.26E+09 | 1.66E+02 |
| 10% | 0.000918 | 2.75E+08 | 5.02E+02 |
| 15% | 0.001 | 1.11E+09 | 1.36E+02 |
| 20% | 0.00171 | 1.00E+08 | 2.57E+03 |
| 30% | 0.000812 | 1.00E+08 | 1.22E+03 |

### 3, Results of Tangential Flow Ultrafiltration Concentration Through Hollow Fiber Columns

The concentration of LRT-mEV raw solution before concentration was 6.0E+12 p/mL, with total particle count of 2.4E+15 particles. After concentration by tangential flow ultrafiltration through hollow fiber columns with 0.5% and 5% PEG3350, the total particle count was higher compared to 0% PEG3350. Under the 5% PEG3350 condition, the highest total particle count reached 1.5E+15, and the concentration reached 7.5E+13 p/mL. This total particle count was 62.5% of the total particle count before concentration, and the concentration was 12.5 times higher than that before concentration. Electron microscopy results showed that the dispersion of mEV was good in the presence of 5% PEG3350.

**Table 3 Concentration Effect of mEV Under Various PEG3350 Concentrations**

| Sample Name | Total particle count after concentration | Particle concentration after concentration |
|---|---|---|
| 0% PEG3350-EV | 2.8E+14 | 1.4E+13 p/mL |
| 0.5% PEG3350-EV | 1.4E+15 | 7.0E+13 p/mL |
| 5% PEG3350-EV | 1.5E+15 | 7.5E+13 p/mL |

### Example 2: Production of High-Concentration mEV Raw Solution Loaded with Semaglutide

Purpose: To load semaglutide (SMT) into mEVs and concentrate the mEV solution using tangential flow ultrafiltration technology while maintaining the stability of particle count and structure of mEVs.

Materials: Milk exosomes (mEVs) prepared with reference to patent 202110097550.X.

### Methods:

1. Loading: See "Example 1" for details.
2. Tangential Flow Ultrafiltration Concentration Through Hollow Fiber Columns (see "Example 1" for details)
3. Measurement of Particle Count: The particle count of the prepared mEV-SMT samples was measured using Flow NanoAnalyzer from NanoFCM (Xiamen).
4. SMT Detection: The SMT polypeptide content in the prepared mEV-SMT samples was measured by LC-MS/MS, and the specific method was referred to the literature (DOI: 10.1016/j.jchromb.2023.123688).

### Results:

### 1. Loading:

Using conditions similar to those for LRT (pH 10.0, no additives), each mEV particle was found to load 20,000 to 40,000 SMT polypeptide molecules.

**Table 4: Loading Capacity of SMT per mEV particle**

| Loaded Sample No. | SMT content (µg) | Total particle count of mEV (particles) | Loading capacity per particle (particles) |
|---|---|---|---|
| 1 | 21.28 | 1.24E+11 | 2.58E+04 |
| 2 | 32.66 | 1.26E+11 | 3.90E+04 |
| 3 | 73.13 | 2.75E+11 | 4.00E+04 |
| 4 | 25.82 | 1.11E+11 | 3.50E+04 |

| | | | |
|---|---|---|---|
| 5 | 20.07 | 1.00E+11 | 3.02E+04 |

The loading capacity of SMT per mEV particle increased with the SMT-to-mEV ratio. However, when the SMT addition amount reached saturation, further increases in SMT addition amount did not enhance loading capacity and could lead to reduced SMT utilization and waste (as shown in Table below). Economy and loading efficiency should be both considered in practical formulation production.

**Table 5: Impact of Initial SMT-to-mEV Ratio on Loading Capacity**

| Corresponding mEV addition amount (p) per SMT addition amount (1.25 mg) | Total particle count of mEV (particles) | SMT addition amount (mg) | SMT loading amount (mg) | SMT utilization rate (%) | Loading capacity per particle (particles) |
|---|---|---|---|---|---|
| 5.00E+10 | 2.10E+12 | 52.50 | 0.57 | 1 | 41000 |
| 1.00E+11 | 1.26E+12 | 15.75 | 0.33 | 2 | 39000 |
| 5.00E+11 | 2.75E+12 | 6.88 | 0.21 | 3 | 11600 |
| 2.00E+12 | 1.11E+12 | 0.69 | 0.02 | 3 | 3170 |
| 1.00E+13 | 1.50E+12 | 0.19 | 0.02 | 10 | 1830 |

### 2. Results of Tangential Flow Ultrafiltration Concentration Through Hollow Fiber Columns

Similar to LRT, using 5% PEG3350 for the concentration of mEV-SMT increased the concentration by at least 10 times compared to before concentration, with the highest final concentration reaching 8E+13p/mL.

**Table 6: Loading Capacity of SMT per mEV particle**

| Concentrated Sample No. | SMT-mEV concentration before concentration (p/mL) | Total particle count of SMT-mEV before concentration (particles) | SMT-mEV concentration after concentration (p/mL) | Total particle count of SMT-mEV after concentration (particles) |
|---|---|---|---|---|
| 1 | 1.60E+12 | 2.56E+15 | 8.20E+13 | 2.46E+15 |
| 2 | 2.70E+12 | 2.70E+15 | 4.90E+ 13 | 2.45E+15 |
| 3 | 4.34E+12 | 4.34E+15 | 7.30E+13 | 3.65E+15 |

### Example 3: Preparation of GLP-1 Polypeptide-mEV Freeze-Dried Orally Disintegrating Tablets

### I. Preparation of LRT-mEV freeze-dried orally disintegrating tablets

Using liraglutide (LRT) loaded into mEV as the starting material, the LRT-mEV freeze-dried orally disintegrating tablets were prepared according to the formulation shown in the table below.

**Table 7: Formulation for LRT-mEV Freeze-Dried Orally Disintegrating Tablets (based on mass before freeze-drying)**

| Batch Number | 220726 | |
|---|---|---|
| Name | Prescribed amount | 100 tablets |
| LRT-mEV raw solution | 200 mg | 20 g |
| Trehalose | 12 mg | 1.2 g |
| Pullulan | 8 mg | 0.8 g |
| Poloxamer 188 | 0.8 mg | 80 mg |
| Xanthan gum | 0.4 mg | 40 mg |
| Purified water | 178.8 mg | 17.88 g |
| Total | 400 mg | 40 g |

(1) The prescribed amounts of LRT-mEV, pullulan, trehalose, and Poloxamer 188 were weighted and placed into a 100 mL beaker. The ingredients were dry mixed and then dissolved in approximately 50% of the prescribed amount of purified water under stirring. The prescribed amount of xanthan gum was weighted and then dissolved in the remaining prescribed amount of purified water under stirring. The two mixtures were then combined.
(2) Shearing: An emulsifier was used for shearing at rotation speed of 300 rpm for 10 minutes.
(3) Degassing: The drug solution was transferred to a 125 ml degassing bottle, and subjected to vacuum degassing until no air bubbles existed.
(4) Filling: The drug solution was filled into 0.4 ml aluminium tray holes using a pipette, 0.4 g/tablet.
(5) Pre-freezing: The filled aluminium tray holes were placed in a low temperature refrigerator at -60°C for 20 minutes of pre-freezing, after which they were kept in this refrigerator until transferred to the freeze dryer.
(6) Freeze-drying Process: The pre-frozen samples were transferred to a 1.7 m² freeze dryer for freeze-drying, and the freeze-drying curve was as follows.
   Start temperature: -30°C, cold trap temperature: ≤-40°C

**Table 8: Freeze-Drying Program**

| Shelf temperature | Heating time | Maintaining time | Vacuum degree |
|---|---|---|---|
| -30°C (Start) | - | - | - |
| -30°C (Vacuum) | - | - | ≤200 ubar |
| -25°C | 5 min | 60 min | ≤200 ubar |
| -15°C | 10 min | 60 min | ≤200 ubar |
| -5°C | 10 min | 60 min | ≤200 ubar |
| 10°C | 25 min | 100 min | ≤200 ubar |
| 25°C | 15 min | 60 min | ≤200 ubar |

**Table 9: Evaluation Results**

| Sample Name: LRT-mEV Freeze-Dried Orally Disintegrating Tablet | | | |
|---|---|---|---|
| Specification: Each tablet contains 200 mg of LRT-mEV raw solution Batch number: 220726 | | | |
| Evaluation basis: "Quality Evaluation Index System for Freeze-dried Flash Release Tablet" (202001 Edition) | | | |
| Item | Evaluation Criterion | Result | Remarks |
| Tablet Appearance | 1-5 (having a smooth and clean surface, uniform color, regular edges, no cracks, no melting or collapse ----- having uneven color, irregular edges, cracks, melting and collapse; tablet breakage: failed, 3 points: passed, 5 points: the best) | 5 | - |
| Tablet cracking | 1-5 | 5 | - |
| Tablet shedding | 1-5 | 5 | - |
| Integrity | 1-5 | 5 | - |
| Mouth feel | 1-5 | 5 | - |
| Taste | 1-5 | 5 | No bitterness |
| Disintegration | 1-5 | 5 | Within 10 seconds |
| Tablet weight difference | 7.5% | 5 | - |
| Result | After evaluation, this product is suitable for development into an orally disintegrating tablet that meets the relevant requirements in the "Quality Evaluation Index System for Freeze-Dried Tablets". | | |

### II. Preparation of SMT-mEV freeze-dried orally disintegrating tablets

Using semaglutide (SMT) loaded into mEV as the starting material, the SMT-mEV freeze-dried orally disintegrating tablets were prepared according to the method and the formulation in I.

**Table 10: Evaluation Results**

| Sample Name: SMT-mEV Freeze-Dried Orally Disintegrating Tablet | | | |
|---|---|---|---|
| Specification: Each tablet contains 200 mg of SMT-mEV raw solution | | | |
| Batch number: 230612 | | | |
| Evaluation basis: "Quality Evaluation Index System for Freeze-dried Flash Release Tablet" (202001 Edition) | | | |
| Item | Evaluation Criterion | Result | Remarks |
| Tablet Appearance | 1-5 (having a smooth and clean surface, uniform color, regular edges, no cracks, no melting or collapse ----- having uneven color, irregular edges, cracks, melting and collapse; | 5 | - |
| | tablet breakage: failed, 3 points: passed, 5 points: the best) | | |
| Tablet cracking | 1-5 | 5 | - |
| Tablet shedding | 1-5 | 5 | - |
| Integrity | 1-5 | 5 | - |
| Mouth feel | 1-5 | 5 | - |
| Taste | 1-5 | 5 | No bitterness |
| Disintegration | 1-5 | 5 | Within 10 seconds |
| Tablet weight difference | 7.5% | 5 | - |
| Result | After evaluation, this product is suitable for development into an orally disintegrating tablet that meets the relevant requirements in the "Quality Evaluation Index System for Freeze-Dried Tablets". | | |

### Example 4: Animal Pharmacodynamic Testing of GLP-1 Polypeptide-mEV Freeze-Dried Orally disintegrating Tablets

Purpose: To evaluate the hypoglycemic effect of GLP-1 polypeptide-mEV freeze-dried orally disintegrating tablets and measure liraglutide blood concentrations using a random blood glucose test in db/db mice.

### I. Method for Pharmacodynamic Testing of LRT-mEV Freeze-Dried Orally disintegrating Tablets

### 1. Animal Pharmacodynamic Test:

a. Experimental Grouping

**Table 11**

| Group | mEV addition amount per tablet | LRT content per tablet (approximation) | Number of animals |
|---|---|---|---|
| A | 2E+13p | 1 mg | 14 (5+9) |
| B | 2E+12p | 1 mg | 14 (5+9) |
| C | 2E+11p | 1 mg | 14 (5+9) |
| Positive control | 5E+11p | 1 mg | 14 (5+9) |
| Negative control | | | 5 |

b. Administration was carried out once at 0 h and once at 7 h.
c. Blood glucose levels were measured at 0 hours in db/db mice. After isoflurane anesthesia, the drug was administered sublingually.
a) Groups A-C: The freeze-dried orally disintegrating tablets were cut into 3-6 small pieces. A small piece was carefully placed under the mouse's tongue, followed by the dropwise addition of 20 µL of water to dissolve the tablet. The administration was repeated in 3 or 6 doses, with a 10-minute interval between each dose for absorption.
b) Positive Control Group: LRT-mEV solution was administered under the mouse's tongue in accordance with the method described in the literature (doi: 10.1002/cmdc.202100758), with a total of 90 µL given in 3 doses, with a 10-minute interval between each dose for absorption.
c) Negative Control Group: No administration

d. Blood glucose levels of mice in each group were measured at 4 h, 7 h, 22 h, 24 h, 26 h, 28 h, and 33 h after the first administration, with each measurement in duplicate. (Mice were allowed to eat and drink during the experimental period, and the random blood glucose was measured.)

### 2. Blood Drug Concentration Measurement

### (1) Blood Collection

Blood was collected from 9 mice each in groups A-C and in the positive control group at different time points (4 h, 22 h and 33 h after the first administration). Three mice in each group were sacrificed at each time point for blood collection.

### (2) Determination of Liraglutide Content in Blood Samples by LC-MS/MS Method

The quantification of liraglutide in blood samples was carried out with reference to the procedure reported in the literature (Shiqi Dong, Yuan Gu, Guangli Wei, Duanyun Si, Changxiao Liu , Determination of liraglutide in rat plasma by a selective liquid chromatography tandem mass spectrometry method: application to a pharmacokinetics study. Chromb (2017), doi:10.1016/j.jchromb.2018. 05.020)

### Results:

### 1. Random Blood Glucose Results of Animal Pharmacodynamics Test

The random blood glucose results of animal pharmacodynamics test showed that blood glucose levels in the positive control group mice decreased significantly within 4 hours after administration and then significantly rebounded within 7 hours. After the second dose, blood glucose levels remained below baseline until recovering to baseline levels at 33 hours. For the orally disintegrating tablet groups (A-C), no significant decrease in blood glucose was observed within 7 hours after the first dose. However, after the second dose, a significant decrease in blood glucose was noted, with the extent and duration of the decrease positively correlated with the number of mEV particles per tablet. Notably, the blood glucose reduction in Group A was greater than that in the positive control group.

### 2. Blood Drug Concentration Results

Blood drug concentration results indicated that in the positive control group, the LRT drug concentration peaked 4 hours after the first dose and then gradually declined. For groups A-C, LRT drug concentration peaked after the second dose, between 7 and 22 hours.

These results suggest that the freeze-dried oral flash release tablets of LRT-mEV dissolve rapidly in the mouse's mouth, and LRT is efficiently absorbed sublingually into the bloodstream, achieving a significant hypoglycemic effect.

### II. Method for Pharmacodynamic Testing of SMT-mEV Freeze-Dried Orally disintegrating Tablets

Purpose: To evaluate the hypoglycemic effect of SMT-mEV freeze-dried orally disintegrating tablets using a random blood glucose test in db/db mice, and to compare the dosing frequency differences between LRT-mEV and SMT-mEV formulations.

### Methods:

### 1. Animal Pharmacodynamic Test 1:

a. Grouping in Experiment 1

**Table 12**

| Group | mEV addition amount per tablet | SMT content per tablet (approximation) | Number of animals |
|---|---|---|---|
| A | 2E+13p | 1 mg | 6 |
| B | 2E+11p | 1 mg | 6 |
| Subcutaneous injection (positive control) | NA | 1 mg | 6 |
| Negative control | | | 5 |

b. Administration was carried out once at 0 h and once at 7 h in oral group, and once at 0 h in subcutaneous injection group.
c. The blood glucose measurement and administration method of db/db mice were the same as before.

### 2. Animal Pharmacodynamic Test 2

a. Grouping in Experiment 2

**Table 13**

| Group | mEV addition amount per tablet | SMT content per tablet (approximation) | Number of animals |
|---|---|---|---|
| LRT-mEV | 2E+13p | 1 mg | 6 |
| SMT-mEV | 2E+13p | 1 mg | 6 |
| Negative control | | | 5 |

b. Administration was carried out twice daily on Day 1-Day 7 in LRT-mEV group and twice on Day 1 in SMT-mEV group, and no more thereafter.
c. The blood glucose measurement and administration method of db/db mice were the same as before.

### Results:

### 1. Random Blood Glucose Results of Animal Pharmacodynamics Test 1

The results of animal pharmacodynamics test 1 showed that oral administration of SMT-mEV orally disintegrating tablets led to a significant decrease in blood glucose levels in db/db mice. This suggests that SMT was effectively absorbed through the oral mucosa when delivered via mEV. Furthermore, formulations with higher mEV content (Group A) demonstrated a faster onset of action compared to those with lower mEV content (Group B), indicating improved absorption efficacy.

### 2. Random Blood Glucose Results of Animal Pharmacodynamics Test 2

The results of animal pharmacodynamics test 2 showed that SMT, as a second-generation long-acting GLP-1 drug, provided sustained hypoglycemic effects for 5-6 days following oral administration of SMT-mEV on the first day in db/db mice. The hypoglycemic effect achieved was comparable to that observed with daily oral administration of LRT-mEV.

## Claims

1. A freeze-dried orally disintegrating tablet of GLP-1 polypeptide drug, **characterized in that**, it comprises, in percentage by mass:
30% to 50% GLP-1 polypeptide-mEV raw solution or GLP-1 polypeptide-LNP raw solution, 0.1% to 1.0% surfactant, 3% to 5% matrix agent, 1% to 3% binder, 0.01% to 1% suspending agent, and water to balance;
the surfactant is selected from poloxamer 188.

2. The freeze-dried orally disintegrating tablet of GLP-1 polypeptide drug according to claim 1, **characterized in that**, the matrix agent is selected from one of mannitol and trehalose; preferably, the matrix agent is trehalose.

3. The detection method according to claim 1, **characterized in that**, the binder is selected from one of gelatin and pullulan; preferably, the binder is pullulan.

4. The freeze-dried orally disintegrating tablet of GLP-1 polypeptide drug according to claim 1, **characterized in that**, the suspending agent is selected from one of xanthan gum and pullulan; preferably, the suspending agent is xanthan gum.

5. The freeze-dried orally disintegrating tablet of GLP-1 polypeptide drug according to claim 1, **characterized in that**, the raw materials for preparing the GLP-1 polypeptide-mEV raw solution comprise: per milligram of GLP-1 polypeptide, 0 to 1.2 mg of an excipient and 1×10¹⁰ to 1×10¹³ mEV particles are added;
the raw materials for preparing the GLP-1 polypeptide-LNP raw solution comprise: per milligram of GLP-1 polypeptide, 0 to 3 mg of an excipient and phospholipid, SM-102 and DLin-MC3-DMA are added;
the excipient is selected from one of poly-lysine, protamine, poly-arginine, fucoidan or mannitol, preferably, the excipient is poly-arginine.

6. The freeze-dried orally disintegrating tablet of GLP-1 polypeptide drug according to claim 1, **characterized in that**, the GLP-1 polypeptide is liraglutide.

7. The freeze-dried orally disintegrating tablet of GLP-1 polypeptide drug according to claims 1-6, **characterized in that**, it comprises the following raw materials in percentage by mass: 30% to 50% liraglutide-mEV raw solution or liraglutide-LNP raw solution, 0.1% to 1.0% poloxamer 188, 3% to 5% trehalose, 1% to 3% pullulan, 0.01% to 1% xanthan gum, and water to balance.

8. The freeze-dried orally disintegrating tablet of GLP-1 polypeptide drug according to claim 1, **characterized in that**, the GLP-1 polypeptide is semaglutide.

9. The freeze-dried orally disintegrating tablet of GLP-1 polypeptide drug according to claims 1-5 or 8, **characterized in that**, it comprises the following raw materials in percentage by mass: 30% to 50% semaglutide-mEV raw solution or semaglutide-LNP raw solution, 0.1% to 1.0% poloxamer 188, 3% to 5% trehalose, 1% to 3% pullulan, 0.01 to 1% xanthan gum, and water to balance.

10. A method for preparing the freeze-dried orally disintegrating tablet of GLP-1 polypeptide drug according to any one of claims 1 to 9, **characterized in that**, it comprises the following steps:
(1) loading GLP-1 polypeptide into mEV to obtain a GLP-1 polypeptide-mEV raw solution, or loading GLP polypeptide into LNP to obtain a GLP-1 polypeptide-LNP raw solution;
(2) concentrating the GLP-1 polypeptide-mEV raw solution or GLP-1 polypeptide-LNP raw solution by ultrafiltration to obtain a concentrated solution,
(3) mixing the surfactant, matrix agent, binder, suspending agent, sweetener and flavoring agent and adding the concentrated solution to obtain a drug solution;
(4) shearing the drug solution in step (3);
(5) degassing the sheared drug solution in step (4);
(6) filling the degassed drug solution in step (5) into a tray hole;
(7) pre-freezing the filled drug solution to obtain a medicinal product; and
(8) freeze-drying the pre-frozen medicinal product with an initial temperature set at -30°C, wherein in the freeze-drying process:
a cold trap temperature is equal to or less than -40°C; a vacuum degree is equal to or less than 200 ubar,
a shelf temperature rises from -30°C to -25°C for 5 minutes, maintains at -25°C for 60 minutes, rises from -25°C to -15°C for 10 minutes, maintains at 15°C for 60 minutes; rises from -15°C to -5°C for 10 minutes, maintains at -5°C for 60 minutes, rises from -5°C to 10°C for 25 minutes, maintains at 10°C for 100 minutes, rises from 10°C to 25°C for 15 minutes, and maintains at 25°C for 60 min.

11. The method according to claim 10, **characterized in that**, the loading of GLP-1 polypeptide into mEV comprises:
mixing a GLP-1 polypeptide solution with an mEV solution, and then performing ultrasonic treatment;
wherein in the GLP-1 polypeptide solution, the solvent is a 20 mM Tris buffer containing 100 mM NaCl, with a pH of 8.0, in the mEV solution, the solvent is a 20 mM Tris buffer containing 100 mM NaCl, with a pH adjusted to 7.0 to 10.0 using a Na₂CO₃/NaHCO₃ buffer containing glycine, and the ultrasonic treatment is carried out at 99% power and 5°C for 12 h.

12. The method according to claim 10, **characterized in that**, the loading of GLP-1 polypeptide into LNP comprises: mixing an aqueous solution of GLP-1 with an organic solvent of lipid molecules, which self-assemble to form an aqueous dispersion of LNP encapsulating GLP-1.

13. The method according to claim 10, **characterized in that**, the concentration of the GLP-1 polypeptide-mEV raw solution or GLP-1 polypeptide-LNP raw solution by ultrafiltration comprises:
dissolving the GLP-1 polypeptide-mEV raw solution or GLP-1 polypeptide-LNP raw solution in PBS buffer containing PEG3350, filtering through a hollow fiber column with a molecular weight cut-off of 750 kDa at a flow rate of 405 mL/min, and concentrating to 1/20th of a volume of the raw solution.

14. The method according to claim 10, **characterized in that**, the shearing is carried out at a rotational speed of 100 to 3000 rpm for 3 to 20 minutes; preferably, the emulsifier operates at a rotational speed of 300 rpm for 10 minutes.

15. The method according to claim 10, **characterized in that**, the pre-freezing is carried out at -60 to -80°C; preferably, the pre-freezing is carried out at -60°C.

16. The method according to claim 10, **characterized in that**, the pre-freezing is carried out for 10 to 100 minutes; preferably, the pre-freezing is carried out for 30 minutes.
